# EUROPÄISCHE PATENTSCHRIFT

(11) **EP 0 889 873 B1**
(45) Veröffentlichungstag und Bekanntmachung des Hinweises auf die Patenterteilung: **25.10.2000**
(21) Anmeldenummer: 97915377.2
(22) Anmeldetag: 18.03.1997
(51) Int. Cl.: C07C 67/26, C07C 69/24, C07C 69/52

(54) **VERFAHREN ZUR HERSTELLUNG ALKOXYLIERTER FETTSÄUREALKYLESTER**
PROCESS FOR PREPARING ALKOXYLATED FATTY ACID ALKYL ESTERS
PROCEDE DE PREPARATION D'ALKYLESTERS D'ACIDE GRAS ALCOXYLES

(30) Priorität: 27.03.1996 DE 19611999
(43) Veröffentlichungstag der Anmeldung: 13.01.1999
(73) Patentinhaber: Cognis Deutschland GmbH, 40589 Düsseldorf (DE)
(72) Erfinder: BEHLER, Ansgar, D-46240 Bottrop (DE); FOLGE, Almud, D-40764 Langenfeld (DE)
(86) Internationale Anmeldenummer: EP9701339
(87) Internationale Veröffentlichungsnummer: WO9735831

(56) Entgegenhaltungen:
- EP-A- 0 339 426
- DE-A- 4 446 064

## Beschreibung

Die Erfindung betrifft ein Verfahren zur Alkoxylierung von Fettsäurealkylestem in Gegenwart von Doppelschichtmischoxiden und ausgewählten Co-Katalysatoren.

### Stand der Technik

Alkoxylierte Alkylester, vorzugsweise sogenannte Methylesterethoxylate, stellen bekannte nichtionische Tenside dar, die wegen ihrer ausgezeichneten Waschleistung in letzter Zeit erheblich an Interesse gewonnen haben. Übersichten hierzu finden sich beispielsweise in **J.Am.Oil.Chem.Soc. 56, 873 (1979) bzw. J.Am.Oil.Chem.Soc. 72, 781 (1995)**.

Die Anlagerung von Alkylenoxiden an Verbindungen mit aciden Wasserstoffatomen, vorzugsweise an primäre Alkohole, gelingt in Gegenwart verschiedenster, in der Regel alkalischer Katalysatoren. Typische Beispiele sind Kaliumhydroxid oder Natriummethylat, die in Form alkoholischer Lösungen zugesetzt werden, oder aber heterogene Schichtverbindungen vom Typ der Hydrotalcite, die man als Feststoffe in die Reaktionsmischung eindosiert. Die Insertion von Alkylenoxiden in die Carbonylesterbindung ist hingegen wesentlich schwieriger und gelingt nur unter Einsatz besonderer Katalysatoren.

Aus den beiden Patentschriften **EP-B1 0 339 426** und **EP-B1 0 523 089** (Henkel) ist die Verwendung von calcinierten bzw. mit Fettsäuren modifizierten Hydrotalciten für die Alkoxylierung von Fettsäureestern bekannt. In der Offenlegungsschrift **DE-A1 44 46 064** (Lion) wird vorgeschlagen, die Ethoxylierung von Methylestern in Gegenwart von Mischmetalloxiden durchzuführen, deren Oberfläche mit Metallhydroxiden bzw. Metallalkoxiden modifiziert worden ist. Diese Verfahren sind jedoch technisch nicht befriedigend, da bei Einsatz von Methylestern stets eine nicht zu vemachlässigende Menge des Ausgangsstoff nichtumgesetzt bleibt und als Nebenprodukte geringe Mengen Methylmonoglycolester entstehen, aus denen infolge Hydrolyse Methylglycol freigesetzt werden kann. Auch die Homologenverteilung ist in vielen Fällen unbefriedigend, da sie kein ausgeprägtes Maximum und zu große Anteile an niedriger- und höherethoxylierten Spezies aufweist.

Die Aufgabe der Erfindung hat somit darin bestanden, neue Katalysatoren zur Verfügung zu stellen, die die Herstellung von alkoxylierten Alkylestem mit eingeengter Homologenverteilung ermöglichen, die frei von nichtumgesetzen Alkylestem und Alkylmonoglycolestern sind.

### Beschreibung der Erfindung

Gegenstand der Erfindung ist ein Verfahren zur Herstellung von alkoxylierten Fettsäurealkylestem der Formel (**I**), 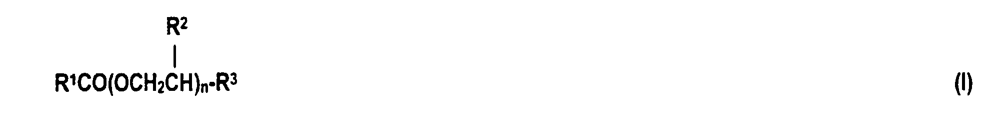 in der R¹CO für einen linearen oder verzweigten Acylrest mit 6 bis 22 Kohlenstoffatomen und 0 und/oder 1, 2 oder 3 Doppelbindungen, R² für Wasserstoff oder eine Methylgruppe, R³ für einen linearen oder verzweigten Alkylrest mit 1 bis 22 Kohlenstoffatomen und n für Zahlen von durchschnittlich 1 bis 20 steht, bei dem man Fettsäureester in Gegenwart von Doppelschichtmischoxid-Katalysatoren mit Ethylen- und/oder Alkylenoxiden umsetzt, das sich dadurch auszeichnet, daß man Co-Katalysatoren einsetzt, die ausgewählt sind aus der Gruppe, die gebildet wird von Lithiumhydroxid, Erdalkalisalzen und Zinnsalzen.

Überraschenderweise wurde gefunden, daß man durch Zusatz der genannten Co-Katalysatoren zu den bekannten Doppelschichtmischoxid-Katalysatoren, insbesondere vom Hydrotalcit-Typ, sowohl den Anteil an nichtumgesetzten Alkylester als auch an Alkylmonoglycolester signifikant vermindert. Zugleich wird eine besonders enge Homologenverteilung erreicht.

### Fettsäurealkylester

Die als Einsatzstoffe in Betracht kommenden Fettsäurealkylester folgen der Formel (**II**),

**R**^{**1**}**CO-OR**^{**3**} **(II)**

in der R¹CO und R³ die oben genannten Bedeutungen besitzen. Die Ester leiten sich von gesättigten und/oder ungesättigten Fettsäuren mit 6 bis 22, vorzugsweise 12 bis 18 Kohlenstoffatomen und Alkoholen mit 1 bis 22, vorzugsweise 1 bis 4 Kohlenstoffatomen ab. Typische Beispiele sind Methyl-, Ethyl-, Propyl-, Butyl- und/oder Stearylester von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technischen Mischungen, die z.B. bei der Druckspaltung von natürlichen Fetten und Ölen oder bei der Reduktion von Aldehyden aus der Roelen'schen Oxosynthese anfallen. Vorzugsweise werden als Einsatzstoffe Kokos- und/oder Talgfettsäuremethylester eingesetzt.

### Katalysatoren

Geeignete Katalysatoren sind beispielsweise die eingangs genannten calcinierten bzw. fettsäuremodifizierten Hydrotalcite wie sie in den europäischen Patenten EP-B1 0 339 426 und EP-B1 0 523 089 ausführlich beschrieben werden; die Lehren dieser beiden Druckschriften wird hiermit ausdrücklich miteingeschlossen. Üblicherweise werden die Katalysatoren in Mengen von 0,1 bis 10 und vorzugsweise 0,5 bis 3 Gew.-% - bezogen auf die Alkoxylierungsprodukte - eingesetzt.

### Co-Katalysatoren

Als Co-Katalysatoren kommen neben dem besonders bevorzugten Lithiumhydroxid Calciumsalze wie beispielsweise Calciumcarbonat, Magnesiumsalze wie beispielsweise Magnesiumoxid, Magnesiumcarbonat oder Magnesiumacetat, Bariumsalze wie beispielsweise Barium-2-ethylhexanoat und/oder Zinnsalze wie bei-spielsweise Zinn(IV)oxid in Frage. Üblicherweise werden die Co-Katalysatoren in Mengen von 0,01 bis 0,5, vorzugsweise 0,1 bis 0,2 Gew.-% - bezogen auf die Alkoxylierungsprodukte-eingesetzt.

### Alkoxylierung

Die Alkoxylierung kann in an sich bekannter Weise durchgeführt werden. Hierzu legt man üblicherweise den Alkylester in einem Druckreaktor mit Rührer vor und setzt den Katalysator zu. Es hat sich als vorteilhaft erwiesen, den Autoklav vor der Reaktion gründlich mit Stickstoff zu spülen, um alle Spuren von Luftsauerstoff zu entfernen. Danach wird der Druckbehälter aufgeheizt, wobei die Alkoxylierung vorzugsweise bei Temperaturen im Bereich von 140 bis 180 und insbesondere von 160 bis 170°C durchgeführt wird. Das Alkylenoxid, bei dem es sich um Ethylenoxid, Propylenoxid oder Mischungen von beiden handeln kann, wird über einen Heber in den Reaktor eingepreßt, wobei der autogene Druck bis auf etwa 5 bar ansteigen kann. Vorzugsweise werden pro Mol Alkylester durchschnittlich 1 bis 20 und insbesondere 8 bis 15 Mol Alkylenoxid, vorzugsweise Ethylenoxid eingesetzt. Die Anlagerung des Alkylenoxids erfolgt dabei statistisch, d.h. bei der Insertion handelt es sich um keine hochselektive Reaktion bei der 1 Mol Fettsäurealkylester mit exakt n Mol Alkylenoxid reagiert. Vielmehr wird ein komplexes Gemisch unterschiedlich hoch alkoxylierter Ester erhalten. Das Ende der Reaktion ist daran zu erkennen, daß der Druck im Reaktor auf etwa 0,5 bar abfällt. Aus Sicherheitsgründen empfiehlt es sich, die Mischung noch weitere 30 min zu rühren, eher der Reaktor abgekühlt und entspannt wird.

### Gewerbliche Anwendbarkeit

Die nach dem erfindungsgemäßen Verfahren erhältlichen alkoxylierten Fettsäurealkylester sind praktisch frei von nichtumgesetzen Alkylestem und Alkylmonoglycolestem. Zudem zeichnen sie sich durch eine besonders vorteilhafte eingeengte Homologenverteilung aus. Sie eignen sich beispielsweise zur Herstellung von Wasch-, Spül- und Reinigungsmitteln.

### Beispiele

**Allgemeine Herstellvorschrift**. In einem 1-1-Rührautoklaven wurden 256 g (1 mol) technischer Laurinsäuremethylester vorgelegt und mit der vorgesehenen Menge Katalysator versetzt. Der Autoklav wurde verschlossen und dreimal abwechselnd mit Stickstoff gespült und evakuiert, um die Anwesenheit von Luftsauerstoff auszuschließen. Anschließend wurde die Reaktionsmischung unter Stickstoffabdeckung auf 165°C erhitzt und portionsweise 528 g (12 mol) Ethylenoxid eindosiert, wobei der autogene Druck zunächst bis auf 3,5 bar anstieg. Die Reaktion wurde fortgeführt, bis der Druck auf 0,5 bar abgesunken war. Nach weiteren 30 min Nachrührzeit wurde der Druckreaktor abgekühlt und entspannt. Als Qualitätskennziffern sind in Tabelle 1 der Gehalt an nichtumgesetztem Methylester (ME), die Summe an unerwünschten niederen Homologen mit 1 bis 5 Mol Ethylenoxideinheiten (ME1/5) sowie der Gehalt an Monomethylglycolester (MGE) angegeben. Die Mengenangaben bei den Katalysatoren und Co-Katalysatoren beziehen sich auf das Endprodukt.

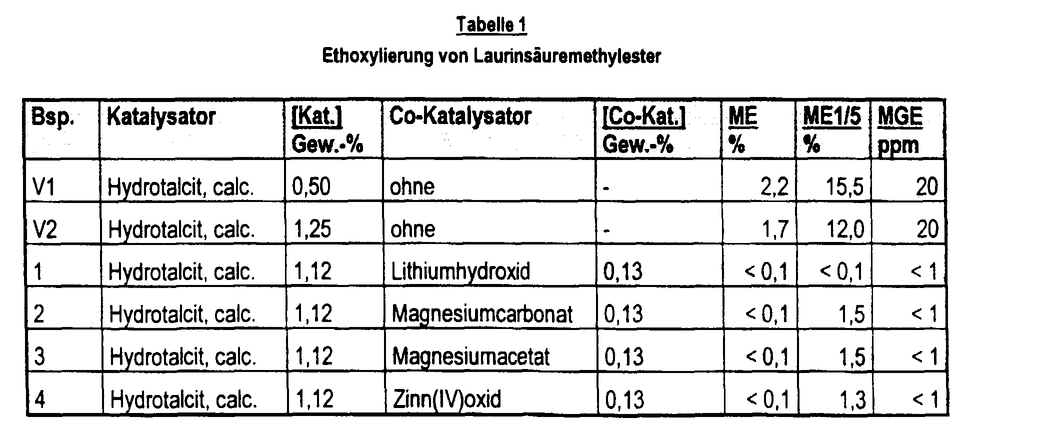

Man erkennt, daß der Zusatz der erfindungsgemäßen Co-Katalysatoren nicht nur den Gehalt an freiem Methylester auf Null bringt, sondern auch die niedrigethoxylierten Homologen signifikant vermindert werden, so daß eine eingeengte Homologenverteilung resultiert. Gleichzeitig wird die Menge an Methylmonoglycolester bis unter die Nachweisgrenze reduziert.

## Patentansprüche

1. Verfahren zur Herstellung von alkoxylierten Fettsäurealkylestem der Formel (**l**), 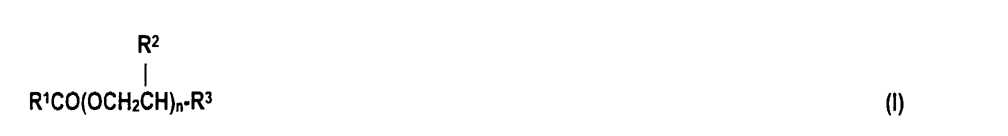 in der R¹CO für einen linearen oder verzweigten Acylrest mit 6 bis 22 Kohlenstoffatomen und 0 und/oder 1, 2 oder 3 Doppelbindungen, R² für Wasserstoff oder eine Methylgruppe, R³ für einen linearen oder verzweigten Alkylrest mit 1 bis 22 Kohlenstoffatomen und n für Zahlen von durchschnittlich 1 bis 20 steht, bei dem man Fettsäureester in Gegenwart von Doppelschichtmischoxid-Katalysatoren mit Ethylen- und/oder Alkylenoxiden umsetzt, **dadurch gekennzeichnet**, daß man Co-Katalysatoren einsetzt, die ausgewählt sind aus der Gruppe, die gebildet wird von Lithiumhydroxid, Erdalkalisalzen und Zinnsalzen.

2. Verfahren nach Anspruch 1, **dadurch gekennzeichnet**, daß man Fettsäurealkylester einsetzt, die der Formel (**II**) folgen,
**R**^{**1**}**CO-OR**^{**3**} **(II)**
in der R¹CO und R³ die oben genannten Bedeutungen besitzen.

3. Verfahren nach den Ansprüchen 1 und 2, **dadurch gekennzeichnet**, daß man als Doppelschichtmischoxid-Katalysatoren calcinierte und/oder mit Fettsäuren hydrophobierte Hydrotalcite einsetzt.

4. Verfahren nach den Ansprüchen 1 bis 3, **dadurch gekennzeichnet**, daß man als Co-Katalysatoren Magnesiumcarbonat, Magnesiumacetat und/oder Zinnoxid einsetzt.

5. Verfahren nach den Ansprüchen 1 bis 4, **dadurch gekennzeichnet**, daß man die Katalysatoren in Mengen von 0,1 bis 10 Gew.-% - bezogen auf die Alkoxylierungsprodukte - einsetzt.

6. Verfahren nach den Ansprüchen 1 bis 5, **dadurch gekennzeichnet**, daß man die Co-Katalysatoren in Mengen von 0,01 bis 0,5 Gew.-% - bezogen auf die Alkoxylierungsprodukte - einsetzt.

7. Verfahren nach den Ansprüchen 1 bis 6, **dadurch gekennzeichnet**, daß man die Alkoxylierung in an sich bekannter Weise unter autogenem Druck bei Temperaturen im Bereich von 140 bis 180°C durchführt.

## Claims

1. A process for the production of alkoxylated fatty acid alkyl esters corresponding to formula (**I**): 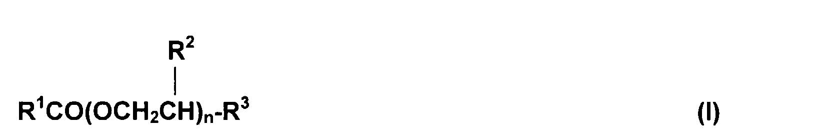 where R¹CO is a linear or branched acyl group containing 6 to 22 carbon atoms and 0 and/or 1, 2 or 3 double bonds, R² is hydrogen or a methyl group, R³ is a linear or branched alkyl group containing 1 to 22 carbon atoms and n is a number of - on average - 1 to 20,
in which fatty acid esters are reacted with ethylene and/or alkylene oxides in the presence of double-layer mixed-oxide catalysts, characterized in that co-catalysts selected from the group consisting of lithium hydroxide, alkaline earth metal salts and tin salts are used.

2. A process as claimed in claim 1, characterized in that fatty acid alkyl esters corresponding to formula (**II**):
**R**^{**1**}**CO-OR**^{**3**} **(II)**
where R¹CO and R³ are as defined above,
are used.

3. A process as claimed in claims 1 and 2, characterized in that calcined hydrotalcites and/or hydrotalcites hydrophobicized with fatty acids are used.

4. A process as claimed in claims 1 to 3, characterized in that magnesium carbonate, magnesium acetate and/or tin oxide is/are used as co-catalysts.

5. A process as claimed in claims 1 to 4, characterized in that the catalysts are used in quantities of 0.1 to 10% by weight, based on the alkoxylation products.

6. A process as claimed in claims 1 to 5, characterized in that the co-catalysts are used in quantities of 0.01 to 0.5% by weight, based on the alkoxylation products.

7. A process as claimed in claims 1 to 6, characterized in that the alkoxylation is carried out in known manner under autogenous pressure at temperatures in the range from 140 to 180°C.

## Revendications

1. Procédé de fabrication d'alkylesters d'acides gras alcoxylés de formule (I), 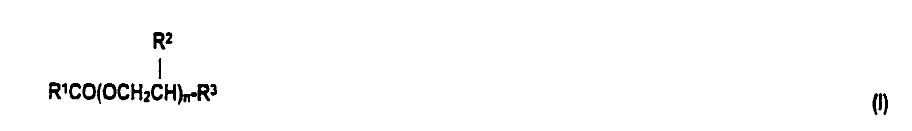 dans laquelle R¹CO représente un radical acyle linéaire ou ramifié comportant de 6 à 22 atomes de carbone et 0 et/ou 1, 2 ou 3 doubles liaisons, R² représente un hydrogène ou un groupe méthyle, R³ représente un radical alkyle linéaire ou ramifié comportant de 1 à 22 atomes de carbone et n représente un nombre en moyenne de 1 à 20, dans lequel on fait réagir des esters d'acides gras en présence de catalyseurs oxydes mixtes en double couche avec des oxydes d'éthylène et/ou d'alkylène,
caractérisé en ce qu'
on emploie des co-catalyseurs choisis dans le groupe formé par l'hydroxyde de lithium, des sels de métaux alcalinoterreux et les sels d'étain.

2. Procédé selon la revendication 1,
caractérisé en ce qu'
on utilise des alkylesters d'acides gras qui répondent à la formule (II)
**R**^{**1**}**CO-OR**^{**3**} **(II)**
dans laquelle R¹CO et R³ possèdent la signification mentionnée ci-dessus.

3. Procédé selon les revendications 1 et 2,
caractérisé en ce qu'
on utilise comme catalyseurs oxydes mixtes en couche double des hydrotalcites calcinées et/ou rendues hydrophobes avec des acides gras.

4. Procédé selon les revendications 1 à 3,
caractérisé en ce qu'
on utilise comme co-catalyseur le carbonate de magnésium, l'acétate de magnésium et/ou l'oxyde d'étain.

5. Procédé selon les revendications 1 à 4,
caractérisé en ce qu'
on utilise les catalyseurs à des quantités de 0,1 à 10 % en poids - par rapport aux produits d'alcoxylation.

6. Procédé selon les revendications 1 à 5,
caractérisé en ce qu'
on utilise les co-catalyseurs à des quantités de 0,01 à 0,5 % en poids - par rapport aux produits d'alcoxylation.

7. Procédé selon les revendications 1 à 6,
caractérisé en ce qu'
on conduit l'alcoxylation de façon connue sous pression autogène à des températures comprises entre 140 et 180°C.
